# EUROPEAN PATENT SPECIFICATION

(11) **EP 4 209 134 B1**
(45) Date of publication and mention of the grant of the patent: **03.12.2025**
(21) Application number: 22214624.3
(22) Date of filing: 19.12.2022
(51) Int. Cl.: A23L 7/104, A23L 13/00, A23L 11/50, A23L 11/60, C12N 1/14, C12R 1/845

(54) **METHOD OF PREPARING FERMENTED FOOD BY USING NOVEL RHIZOPUS MICROSPORUS STRAIN, AND FERMENTED FOOD**
VERFAHREN ZUR HERSTELLUNG EINES FERMENTIERTEN LEBENSMITTELS UNTER VERWENDUNG EINES NEUARTIGEN RHIZOPUS-MIKROSPORUS-STAMMS UND FERMENTIERTES LEBENSMITTEL
PROCÉDÉ DE PRÉPARATION D'UN ALIMENT FERMENTÉ À L'AIDE D'UNE NOUVELLE SOUCHE DE RHIZOPUS MICROSPORUS, ET ALIMENT FERMENTÉ

(30) Priority: 29.12.2021 TW 110149312
(43) Date of publication of application: 12.07.2023
(73) Proprietor: Industrial Technology Research Institute, Chutung, Hsinchu 310401 (TW)
(72) Inventor: Cheng, Chun-Shen, Kaohsiung City, Taiwan (R.O.C.) (TW); Fan, Chih-Hsuan, Hsinchu City, Taiwan (R.O.C.) (TW); Tsai, Shu-Hsien, Kaohsiung City, Taiwan (R.O.C.) (TW); Chien, Chuan-Chi, Kaohsiung City, Taiwan (R.O.C.) (TW); Lee, Shih-Chi, Pingtung, Taiwan (R.O.C.) (TW); Cheng, Hsiang Tsai, Kaohsiung City, Taiwan (R.O.C.) (TW); Huang, Yu Lung, Kaohsiung City, Taiwan (R.O.C.) (TW)
(74) Representative: Fuchs Patentanwälte Partnerschaft mbB

(56) References cited:
- CN-A- 113 025 500
- ERKAN SELIME BENEMIR ET AL: "Production and characterization of tempehs from different sources of legume by Rhizopus oligosporus", LWT- FOOD SCIENCE AND TECHNOLOGY, ACADEMIC PRESS, UNITED KINGDOM, vol. 119, 25 November 2019 (2019-11-25), XP085975428, ISSN: 0023-6438, [retrieved on 20191125], DOI: 10.1016/J.LWT.2019.108880
- CHEN-TIEN CHANG ET AL: "Effect of fermentation time on the antioxidant activities of tempeh prepared from fermented soybean using Rhizopus oligosporus", INTERNATIONAL JOURNAL OF FOOD SCIENCE AND TECHNOLOGY, BLACKWELL SCIENTIFIC PUBLICATIONS, OXFORD, GB, vol. 44, no. 4, 13 March 2009 (2009-03-13), pages 799 - 806, XP071855413, ISSN: 0950-5423, DOI: 10.1111/J.1365-2621.2009.01907.X
- BARUS TATI ET AL: "Genotypic Characterization of Rhizopus species from Tempeh and Usar: Traditional Inoculum of Tempeh in Indonesia", MICROBIOLOGY INDONESIA, vol. 14, no. 3, 1 January 2020 (2020-01-01), pages 101 - 107, XP093050924, ISSN: 1978-3477, Retrieved from the Internet <URL:https://jurnal.permi.or.id/index.php/mionline/article/download/742/657> DOI: 10.5454/mi.14.3.3

## Description

### Technical Field

The disclosure relates to an isolated and purified novel *Rhizopus microsporus* strain, a method for preparing a fermented food by using the isolated and purified novel *Rhizopmicrosporus* strain, and a fermented food prepared thereby.

### Background

For a long time, meat has been the main source of protein for human beings. With the growth of the economy and the increase of the global population, the demand for meat products is increasing. However, the carbon emissions produced from meat production are quite high, and the excrement of many livestock emits large amounts of greenhouse gases. In particular, when ruminants (such as cattle and sheep) ruminate, a large amount of methane gas is produced in the intestines. The effect of methane on global warming is about 20 times higher than that of carbon dioxide, which has a great impact on exacerbating the climate crisis. The preparation of fermented products is known in the art: CN 113025500 A discloses a *rhizopus oligosporus* CGMCC NO.21461 and provides application of the *rhizopus oligosporus* in the preparation of fermented products and a method for preparing a bean dreg fermented product by using the *rhizopus oligosporus.* The *rhizopus oligosporus* has capability of degrading proteins and peptides in raw materials in the fermentation process and is a strain for preparing fermented products. The nutritional quality of the bean dreg fermented product prepared by using the *rhizopus oligosporus* as a fermentation strain is improved. Erkan et al. (LWT - Food Science and Technology 119 (2020) 108880; Production and characterization of tempehs from different sources of legume by *Rhizopus oligosporus*) report a study to determine the physical-chemical features of tempehs produced from different sources of legume (soybean (control), chickpea, lentils (red and green), and beans (white, black, and broad)) using *Rhizopus oligosporus* and to specify the best tempeh by sensory evaluation. Chang et al. (International Journal of Food Science and Technology 44, 2009, 799-806, Effect of fermentation time on the antioxidant activities of tempeh prepared from fermented soybean using *Rhizopus oligosporus*) report a study to evaluate the effect of fermentation time on the antioxidant activity of tempeh, a fermented product from soybean. Chang et al. disclose the use of BCRC 31750, a *Rhizopus oligosporus* strain, to prepare tempeh. Barus et al. (Microbiology Indonesia ISSN 1978-3477, elSSN 2087-8575, 14, 2020, 101-107, Genotypic Characterization of *Rhizopus* species from Tempeh and *Usar:* Traditional Inoculum of Tempeh in Indonesia) discloses *Rhizopus microsporus* isolates from tempeh in Indonesia.

In recent years, due to people's pursuit of a healthier diet and the increased attention given to animal welfare and environmental friendliness, the issue of seeking alternative sources of protein other than meat and diversifying protein sources has increasingly attracted worldwide attention. Alternative protein sources include, for example, plant-based protein, insect-derived protein, artificial meat developed using bioengineering technology, and protein produced by fungal fermentation etc. However, further development of alternative proteins with high nutritional value is still one of the research goals in related fields.

### SUMMARY

According to embodiments of the disclosure, an isolated and purified novel *Rhizopus microsporus* strain is provided. The deposit number of the isolated and purified novel *Rhizopus microsporus* strain is DSM 34400.

According to embodiments of the disclosure, a method for preparing a fermented food by using a *Rhizopus microsporus* strain is provided. The method includes the following steps: providing an isolated and purified *Rhizopus microsporus* strain, and its deposit number is DSM 34400; and inoculating the isolated and purified *Rhizopus microsporus* strain to a substrate for fermentation to form a fermented food, and the substrate includes a legume, a processing residue of a legume, or a combination thereof.

According to embodiments of the disclosure, a fermented food is provided. The fermented food is formed by fermentation of a substrate by using an isolated and purified *Rhizopus microsporus* strain. The deposit number of the isolated and purified *Rhizopus microsporus* strain is DSM 34400. The substrate includes a legume, a processing residue of a legume, or a combination thereof. In addition, the fermented food has a hardness of 6.48 N to 27.54 N, a springiness of 0.24 Nm to 0.70 Nm, a chewiness of 0.70 N to 5.00 N, a gumminess of 2.7 N to 10.05 N, a cohesiveness of 0.32 to 0.80, and a resilience of 0.14 to 0.50.

A detailed description is given in the following embodiments with reference to the accompanying drawings.

### BRIEF DESCRIPTION OF THE DRAWINGS

FIGs. 1A to 1C show the process of screening the filamentous single strain Tp-1 from the colonies on the medium in according to embodiments of the disclosure.
FIG. 2 shows the alignment result of the sequence of strain Tp-1 (SEQ ID NO: 1) and the sequence of *Rhizopus microspores* isolate TB55 using BLAST in according to embodiments of the disclosure.
FIG. 3 shows the growth curve diagram (relation between culture time and area percentage) of strain Tp-1 when growing on a PDA medium in according to embodiments of the disclosure.
FIG. 4 shows the growth curve diagram (relation between culture time and area percentage) of strain Tp-1 when growing on a PDA medium containing different proportions of bean dregs in according to embodiments of the disclosure.
FIG. 5 shows comparison of photographs taken before and after culture of strain Tp-1 in PDB medium containing different proportions of bean dregs in according to embodiments of the disclosure.

### DETAILED DESCRIPTION

The isolated and purified novel *Rhizopus microsporus* strain, the method for preparing fermented food by using the *Rhizopus microsporus* strain, and the fermented food of the present invention are described in detail in the following description. It should be understood that in the following detailed description, for purposes of explanation, numerous specific details and embodiments are set forth in order to provide a thorough understanding of the present disclosure. The specific elements and configurations described in the following detailed description are set forth in order to clearly describe the present disclosure. It will be apparent that the exemplary embodiments set forth herein are used merely for the purpose of illustration and not the limitations of the present disclosure.

In the following description, the terms "about" and "substantially" typically mean +/- 10% of the stated value, or typically +/- 5% of the stated value, or typically +/- 3% of the stated value, or typically +/- 2% of the stated value, or typically +/- 1% of the stated value or typically +/- 0.5% of the stated value. The stated value of the present disclosure is an approximate value. When there is no specific description, the stated value includes the meaning of "about" and "substantially". In addition, the term "in a range from the first value to the second value" or "in a range between the first value and the second value" means that the range includes the first value, the second value, and other values in between.

Unless defined otherwise, all technical and scientific terms used herein have the same meaning as commonly understood by one of ordinary skill in the art to which this disclosure belongs. It should be appreciated that, in each case, the term, which is defined in a commonly used dictionary, should be interpreted as having a meaning that conforms to the relative skills of the present disclosure and the background or the context of the present disclosure, and should not be interpreted in an idealized or overly formal manner unless so defined.

According to embodiments of the invention, a novel *Rhizopus microsporus* strain was isolated and purified from the banana leaves of the banana planted in Qishan, Kaohsiung, Taiwan. The *Rhizopus microsporus* strain has been deposited in the Deutsche Sammlung von Mikroorganismen und Zellkulturen GmbH (DSMZ), and its deposit number is DSM 34400. The *Rhizopus microsporus* strain also has been deposited in the Food Industry Research and Development Institute, and its deposit number is BCRC 930226. Furthermore, in according to embodiments of the disclosure, by using beans or their processing residues as a culture substrate, and using the aforementioned *Rhizopus microsporus* strain to carry out a fermentation process, the fermented food produced has an improved protein content and is easy to digest in human intestinal tract. Moreover, the fermented food produced is also easy to process into a textured vegetable protein (TVP) additive. In addition, the fermented food can serve as a plant-based protein to replace livestock, poultry and seafood. The fermented food has the characteristics of low calorie, low cholesterol and high-quality protein, providing a diversified source of healthy food, and it also contributes to a considerable degree of net zero carbon emissions for the environment.

As described above, an isolated and purified novel *Rhizopus microsporus* strain is provided in the present invention, whose deposit number is DSM 34400 (deposited in the Deutsche Sammlung von Mikroorganismen und Zellkulturen GmbH on September 29, 2022); and BCRC 930226 (deposited in the Bioresource Collection and Research Center of the Food Industry Research and Development Institute on September 23, 2021). Specifically, this strain was obtained from banana leaves in the banana production area of Qishan, Kaohsiung, Taiwan through the steps of microbial screening, cultivation, isolation and purification. This strain has been identified as *Rhizopus microsporus* TB55 strain. In embodiments not according to the claimed invention, the nucleotide sequence of the isolated and purified novel *Rhizopus microsporus* strain has at least about 95% similarity with the sequence shown in SEQ ID NO: 1, for example, they may have 96%, 97%, 98% or 99% similarity. The nucleotide sequence of the isolated and purified novel *Rhizopus microsporus* strain is shown in SEQ ID NO: 1.

A method for preparing a fermented food by using a *Rhizopus microsporus* strain is also provided in the present disclosure. The method includes the following steps: (a) providing the aforementioned isolated and purified *Rhizopus microsporus* strain, and its deposit number is DSM 34400 (BCRC 930226); and (b) inoculating the isolated and purified *Rhizopus microsporus* strain to a substrate for fermentation to form a fermented food. In addition, the substrate may include, but is not limited to, a legume, a processing residue of a legume, another suitable substrate, or a combination thereof.

In accordance with some embodiments, the isolated and purified *Rhizopus microsporus* strain is provided in the form of a freeze-dried powder or a bacterial broth.

In accordance with some embodiments, the legume may include soybean, black bean, red bean, mung bean, edamame, kidney bean, red kidney bean, pinto bean, pea, pigeon pea, cowpea, broad bean, chickpea, lentil, hyacinth bean, peanut, lupine, grass pea, carob, or a combination thereof, but it is not limited thereto.

In accordance with some embodiments, the processing residue of the legume may include dregs of the legumes, but it is not limited thereto. In accordance with some embodiments, a processing may be performed on the legume to obtain the aforementioned processing residue, and the processing may include a cooking step, a pressing step, a drying step, a grinding step, or a combination thereof, but it is not limited thereto. In accordance with some embodiments, the water content of the processing residue may be less than about 15%, for example, less than 14%, less than 13%, less than 12%, less than 11%, less than 10%, less than 9%, less than 8%, less than 7%, less than 6% or less than 5%.

In accordance with some embodiments, the substrate used for fermentation may be the dregs of legumes, i.e. bean dregs (okara). Bean dregs are by-products of the processing of beans, and the processing of beans usually produces a large amount of bean dregs. Bean dregs contain many functional components, such as isoflavone, phenolic acid and phytosterol etc. However, bean dregs can usually only be discarded or used as feed or fertilizer due to the high moisture content during production, which can easily lead to spoilage and odor. In particular, in accordance with the embodiments of the present disclosure, bean dregs can be reused in a circular economy manner, the protein content and nutritional value of bean dregs can be increased through the fermentation process, and the amount of discarded bean dregs can be reduced and the load on the environment can be reduced.

In accordance with some embodiments, the fermentation of the *Rhizopus microsporus* strain in the substrate may include a solid-state fermentation process, a liquid-state fermentation process or a combination thereof. In accordance with some embodiments, the temperature of the fermentation may be between about 20°C and about 40°C, e.g., 25°C, 30°C, or 35°C. In accordance with some embodiments, the fermentation may be performed for between about 20 hours and about 50 hours, or between about 24 hours and about 48 hours, e.g., 28 hours, 32 hours, 36 hours, 40 hours, or 44 hours.

In accordance with some embodiments, after the isolated and purified *Rhizopus microsporus* strain is inoculated to the substrate to carry out fermentation, the substrate may be further subjected to a uniaxial wet extrusion process or a biaxial wet extrusion process, to form the fermented food. In accordance with some embodiments, the fermented food prepared by the foregoing method may include tempeh, a texturized vegetable protein (TVP) additive, a functional beverage, or a combination thereof, but it is not limited thereto. In accordance with some embodiments, the fermentation broth produced by the fermentation can directly serve as a functional beverage.

In addition, the embodiments of the disclosure also provide a fermented food. The fermented food is formed by fermentation of a substrate by using the aforementioned isolated and purified *Rhizopus microsporus* strain. The hardness of the fermented food may be between about 6.48 N to about 27.54 N. The springiness of the fermented food may be between about 0.24 Nm to about 0.70 Nm. The chewiness of the fermented food may be between about 0.7 N to about 5.00 N. The gumminess of the fermented food may be between about 2.7 N to about 10.05 N. The cohesiveness of the fermented food may be between about 0.32 and about 0.80. The resilience of the fermented food may be between about 0.14 and about 0.50. In accordance with some embodiments, the aforementioned parameters such as hardness, springiness, chewiness, gumminess, cohesiveness, and resilience are the measurement results obtained by a texture analyzer (Horn Instruments co., ltd., model: UniversalTA).

As described above, the substrate may include, but is not limited to, a legume, a processing residue of a legume, another suitable substrate, or a combination thereof.

In accordance with some embodiments, the legume may include soybean, black bean, red bean, mung bean, edamame, kidney bean, red kidney bean, pinto bean, pea, pigeon pea, cowpea, broad bean, chickpea, lentil, hyacinth bean, peanut, lupine, grass pea, carobs, or a combination thereof, but it is not limited thereto.

In accordance with some embodiments, the processing residue of the legume may include dregs of the legumes, but it is not limited thereto. Furthermore, in accordance with some embodiments, the fermented food may include tempeh, a texturized vegetable protein (TVP) additive, a functional beverage, or a combination thereof, but it is not limited thereto.

It should be noted that the fermented food prepared by the method for preparing fermented food by using the *Rhizopus microsporus* strain provided by the embodiments of the present disclosure can have improved content of protein and water-soluble dietary fiber, and can regulate blood sugar, lower cholesterol, delay the absorption of glucose in the small intestine, and enhance intestinal peristalsis. In addition, the resulting fermented food (e.g., tempeh) can improve the digestion and absorption rate of protein, making it easy to digest and not easy to cause flatulence, thereby meeting the protein needs of the elderly, the infirm, and those with weak digestive systems.

Specifically, in accordance with some embodiments, the protein content of the resulting fermented food may be increased by about 2% to about 10% compared to that of the unfermented substrate. Specifically, in accordance with some embodiments, the protein content of the resulting fermented food may be between about 22% to about 28%, based on the total weight of the fermented food. In accordance with some embodiments, the water-soluble dietary fiber content of the resulting fermented food may be between about 2.70% to about 5.67%, based on the total weight of the fermented food.

In order to make the above-mentioned and other purposes, features and advantages of the present disclosure more thorough and easy to understand, a number of examples and comparative examples are given below, and are described in detail as follows, but they are not intended to limit the scope of the present disclosure.

### Example 1: The isolation, purification and identification of novel Rhizopus microsporus strain

The banana leaves planted in the Qishan area of Kaohsiung, Taiwan were collected. After cleaning and drying, cover the peeled soy beans with the banana leaves for fermentation and growth. The filamentous fungi attached to banana leaves were taken, followed by culture, screening, isolation, purification and identification.

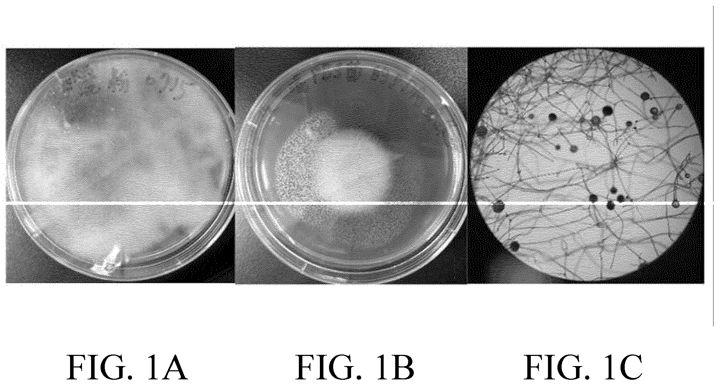

First, 100 µl of the broth (Potato dextrose broth, STBIO MEDIA, INC.) for culturing the filamentous fungi were taken, and 900 µl of secondary water was added and coated on a PDA medium plate (Potato Dextrose Agar Broth; Becton, Dickinson and Company, USA) and incubated at 30°C for 24 hours. Afterwards, the colonies on the medium were picked and again streaked on the same PDA medium plate for strain purification. After culturing at 30°C for 24 hours, the hyphae were picked out, stained using the Gram stain method and observed with electron microscope, and several rounds of screening were repeated until a single bacterial phase strain was observed by electron microscope. Refer to FIGs. 1A to 1C, which show the process of screening the filamentous single strain (strain number Tp-1) from the colonies on the medium. Next, 20% glycerol was added to the isolated and purified strain, it was cryopreserved at - 80°C, and a portion of the strain was taken for the following nucleic acid identification.

According to the instructions of the QIAGEN DNeasy Plant Kit, the DNA of the aforementioned strain was extracted. Sequencing of the internal transcribed spacer (ITS) was performed by polymerase chain reaction using the fungal universal primer pair ITS4 (as shown in SEQ ID NO: 2) and ITS5 (as shown in SEQ ID NO: 3) (MISSION BIOTECH CO., LTD.). Applied Biosystem^{™} (ABI) BigDye Terminator v3.1 Cycle Sequencing Kit and the sequencing machine ABI 3730XL DNA Analyzer were used to sequence the DNA extraction sample of strain Tp-1, and the obtained nucleotide sequence was as shown in SEQ ID NO: 1.

Next, alignment of the nucleotide sequence of SEQ ID NO: 1 and the known biological sequence database (National Center for Biotechnology Information, NCBI) was performed using BLAST (Basic Local Alignment Search Tool). The program aligns nucleotide or protein sequences with the sequence database and calculates statistical significance, combined with verification of physiological and biochemical characteristics. It was identified that the aforementioned isolated and purified strain Tp-1 was more than 99% similar to the *Rhizopus microspores* isolate TB55 in the database.

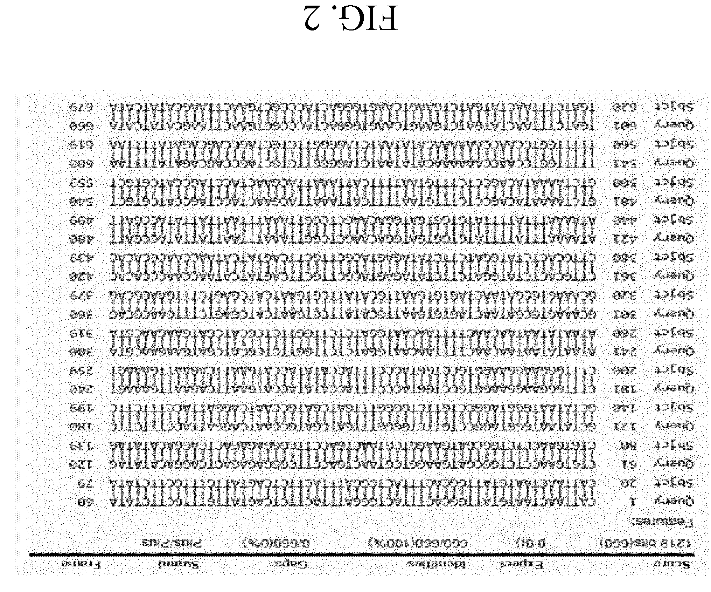

The sequence alignment result of SEQ ID NO: 1 and *Rhizopus microspores* isolate TB55 (gene bank: MF445290.1) is as shown in FIG. 2. The sequencing read length of TB55 is 695bp, 660 bp of the sequence is the same as the SEQ ID NO: 1, and 35 bp of the sequence is different from the SEQ ID NO: 1. Therefore, it can be determined that the isolated and purified strain Tp-1 belongs to the TB55 strain of *Rhizopus microsporus,* but the Tp-1 and the TB55 are different strains.

After the identification was completed, the isolated and purified novel *Rhizopus microsporus* strain Tp-1 was deposited and preserved in the Deutsche Sammlung von Mikroorganismen und Zellkulturen GmbH, and the deposit number was DSM 344006. The isolated and purified novel *Rhizopus microsporus* strain Tp-1 was also deposited and preserved in the Bioresource Collection and Research Center of the Food Industry Research and Development Institute, and the deposit number was BCRC 930226.

### Example 2: Plotting and analysis of the growth curve of novel Rhizopus microsporus strain

An appropriate amount of mold spores was collected from the Tp-1 strain sample to a PDA medium plate (Difco^{™} Potato dextrose agar) using an inoculation loop, cultured at 30°C, and photographed at regular intervals (8 to 12 hours). Since the morphology of *Rhizopus* is filamentous, it grows in a staggered and irregular shape on the solid PDA medium plate. Therefore, the area percentage calculation method was used for quantitative analysis, and the growth curve was plotted using the relation between the area percentage of the growth strain and the time. The life cycle of the strain Tp-1 in the fermentation process can be known, thereby assessing the conditions of subsequent fermentation (e.g., the fermentation time).

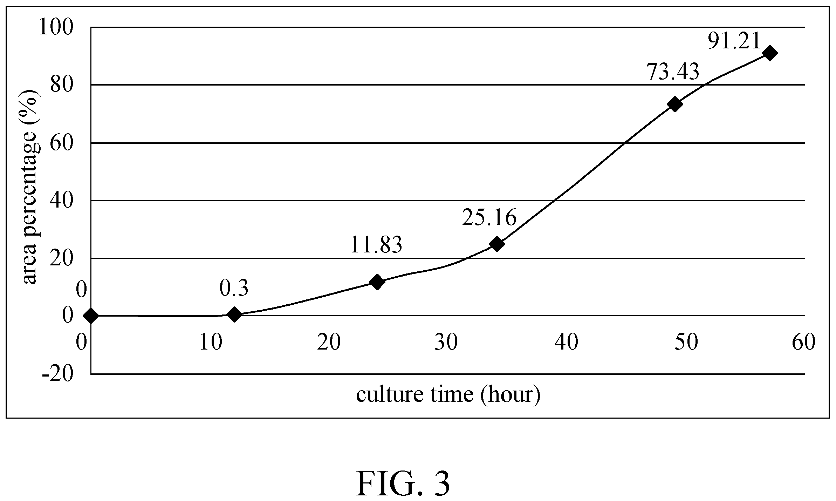

Refer to FIG. 3, which shows the growth curve diagram (relation between culture time and area percentage) of the strain Tp-1 when growing on a PDA medium plate. As shown in FIG. 3, after about 30 hours of culture, the growth rate of the strain Tp-1 began to increase, and the period of about 34 to 49 hours of culture was the rapid growth period of the strain Tp-1. After about 50-57 hours of culture, the growth gradually slowed down, the area percentage of Tp-1 growing on the medium plate was over 90%, and the distribution of white hyphae on the medium was close to saturation. From the growth analysis of the strain Tp-1, it can be seen that the strain Tp-1 is suitable for the production of the solid-state fermentation process.

### Example 3: The growth and functional test analysis of novel Rhizopus microsporus strain by addition of bean processing residues

An appropriate amount of mold spores was collected from the Tp-1 strain sample to a PDA medium plate (Difco^{™} Potato dextrose agar) containing 0%, 5%, 6% and 7% (w/v%) bean dregs respectively using an inoculation loop, cultured at 30°C, and photographed at regular intervals (8 to 12 hours). The area percentage calculation method was used for quantitative analysis, and the growth curve was plotted using the relation between the area percentage of the growth strain and the time.

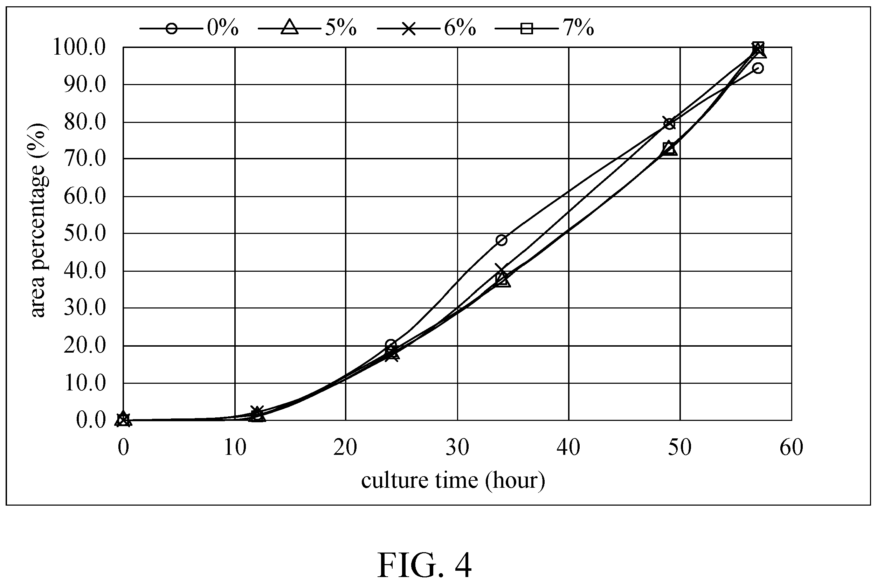

Refer to FIG. 4, which shows the growth curve diagram (relation between culture time and area percentage) of strain Tp-1 when growing on a PDA medium containing different proportions of bean dregs. As shown in FIG. 4, the overall growth trends of the strain Tp-1 in the mediums containing bean dregs were better than the medium without bean dregs, and the growth rates of the strain Tp-1 in the mediums containing bean dregs were faster. Furthermore, the growth rate of the strain Tp-1 in the medium containing 6% bean dregs was the fastest, while the growth rates of the stain Tp-1 in the medium containing 5% and 7% bean dregs were similar at all stages. From the above results, it can also be known that the strain Tp-1 prefers the medium with more protein content.

### Example 4: The composition analysis of the fermentation product of novel Rhizopus microsporus strain

An appropriate amount of mold spores was collected from the Tp-1 strain sample to PDB broth (Potato dextrose broth, STBIO MEDIA) containing 0%, 5%, 6% and 7% (w/v%) bean dregs respectively using an inoculation loop. The fermentation culture was performed at 30°C and 120 rpm for 4 days. After the culture was completed, the culture medium was centrifuged at 20°C and 9900 rpm for 40 minutes, and the supernatant and the precipitate were stored separately. Next, the precipitate was freeze-dried for 2 days and ground into powder, and the crude protein, crude fat, carbohydrate, water-soluble dietary fiber and water-insoluble dietary fiber content of the fermented product were analyzed. The measurement methods were as follows. Experimental results are shown in Table 1.

### 1. Analysis of crude protein content

The total nitrogen content was determined using the Kjeldahl nitrogen method. 0.5 g of the sample was taken and added together with 5 g of catalyst (K₂SO₄:CuSO₄^{·}H₂O = 9:1) and 13 mL of 18N concentrated sulfuric acid to a decomposition tube, and it was put in a protein decomposition furnace, heated to 200°C for 1 hour. After the sample was carbonized into a black liquid, it was heated to 400°C for 2 hours. Until decomposed into a light blue and clear state, it was taken out and cooled down, and then 70 mL of distilled water and 50 mL of 40% NaOH were added. Nitrogen was distilled out with a nitrogen distiller, and a 4% boric acid solution containing methyl blue and methyl red indicators received the distilled nitrogen to make the liquid turn red. Finally, 0.1N HCl was used for titration until the liquid turned to slightly red (that is, the titration is completed), and the amount of titrated liquid was recorded. The crude protein content (%) = [hydrochloric acid titration amount (ml) × hydrochloric acid concentration (N) × 1.4007 × 1] / sample weight (g) × nitrogen content factor (nitrogen conversion factor) 6.25.

### 2. Analysis of crude fat content

3 g of the sample was taken in the filter paper, and it was placed in a filter thimble after wrapping. It was then put into a Soxhlet extractor connected with a fat collection bottle, and 170 mL of n-hexane was added from the upper end of the extractor. After the extractor was connected to a condenser tube, reflux in a constant temperature water bath at 50-60°C for 16 hours. After the extraction, the fat collection bottle was removed, and the n-hexane in the Soxhlet extractor was recovered. The fat collection bottle was placed in an oven at 105°C to dry, taken out and placed in a drying dish to cool until the fat collection bottle had a constant weight. Crude fat (%) = [(weight of fat collection bottle after fat extraction - fat collection bottle weight)]/sample weight × 100%.

### 3. Analysis of total carbohydrate content

The total carbohydrate content is obtained by subtracting the percentage of moisture, ash, crude protein and crude fat from 100%.

### 4. Analysis of water-soluble dietary fiber content

According to the above-mentioned extraction process, the water-insoluble dietary fiber was separated from the filtrate, and the filtrate was poured into a beaker with 300mL of 95% alcohol, so that the water-soluble dietary fiber was precipitated, and it was placed for one day for complete precipitation. The filter paper was weighed and recorded to the fourth decimal place. The liquid was slowly poured into a magnetic funnel for suction filtration, rinsed with 20 mL of 78% alcohol three times, then rinsed with 10 mL of 95% alcohol twice, and finally rinse with 10 mL of acetone twice. The filter and residues were put into a weighing bottle together, put into a 105°C oven and dried to constant weight, and the weight of the weighing bottle and filter paper was subtracted from the constant weight to obtain a total residue weight. Water-soluble dietary fiber content (%) = [total residue net weight - protein weigh in the sample (g) - ash weight in the sample (g)]/sample weight (g) × 100%.

### 5. Analysis of water-insoluble dietary fiber content

The constant temperature water bath was preheated to 95°C, and about 1 g of powdery sample was weighed and recorded to the fourth decimal place. The sample was put into a beaker, 50 mL of phosphate buffer was added and mixed evenly, and then 100 µl of α-amylase was added and shaken lightly. The beaker was covered with aluminum foil, put into a 95°C constant temperature water bath, shaken gently every 5 minutes for about 15 minutes, cooled to room temperature, and then 10 mL of 0.275N NaOH was added and shaken gently. The constant temperature water bath was preheated to 60°C, 0.05 g of protease was added to 1 mL of phosphate buffer, mixed evenly, and 100 µl of protease phosphate buffer solution was added to the beaker. The beaker was covered with aluminum foil, and put into a 60°C constant temperature water bath, shaken gently every 5 minutes for about 30 minutes, cooled to room temperature, and then 10mL of 0.325N HCl was added and shaken gently. The constant temperature water bath was preheated to 60°C, and 100µl of amyloglucosidase was added to the beaker. The beaker was covered with aluminum foil, and put into a 60°C constant temperature water bath, shaken gently every 5 minutes for about 30 minutes, and then cooled to room temperature. The filter paper was weighed and recorded to the fourth decimal place, and the liquid was slowly poured into a magnetic funnel for suction filtration. The filter and residues were put into a weighing bottle together, put into a 105°C oven and dried to constant weight, and the weight of the weighing bottle and filter paper was subtracted from the constant weight to obtain a total residue weight. Water-insoluble dietary fiber content (%) = [total residue net weight - protein weight in the sample (g) - ash weight in the sample (g)]/sample weight (g) × 100%.

**Table 1**

| | 0% bean dregs /PDB | 5% bean dregs /PDB | 6% bean dregs /PDB | 7% bean dregs /PDB |
|---|---|---|---|---|
| Crude protein (%) | 23.75±0.11 | 26.58±0.83 | 26.82±0.66 | 27.47±0.34 |
| Crude fat (%) | 14.31±1.75 | 12.49±1.33 | 12.23±1.74 | 13.23±3.06 |
| Carbohydrate (%) | 55.00±2.24 | 51.33±1.15 | 51.26±2.63 | 48.85±3.51 |
| Water-soluble dietary fiber (%) | 1.72±0.95 | 4.77±0.89 | 3.18±0.63 | 3.50±0.14 |
| Water-insoluble dietary fiber (%) | 21.70±1.80 | 18.97±2.38 | 17.92±0.80 | 17.35±4.30 |

According to the results of Table 1, after Tp-1 was inoculated in the medium of containing different proportions of bean dregs (0%, 5%, 6%, 7% (w/v%)) and fermented for 96 hours, the crude protein content of the mediums containing the bean dregs increased. Compared with the group without bean dregs, the protein content of the group containing 7% bean dregs increased by about 4%, the glycolysis conversion rate was about 6%, and the water-soluble dietary fiber increased by about 2.1%.

### Comparative Example 1: The composition analysis of the fermentation product of BCRC 31750 tempeh bacteria

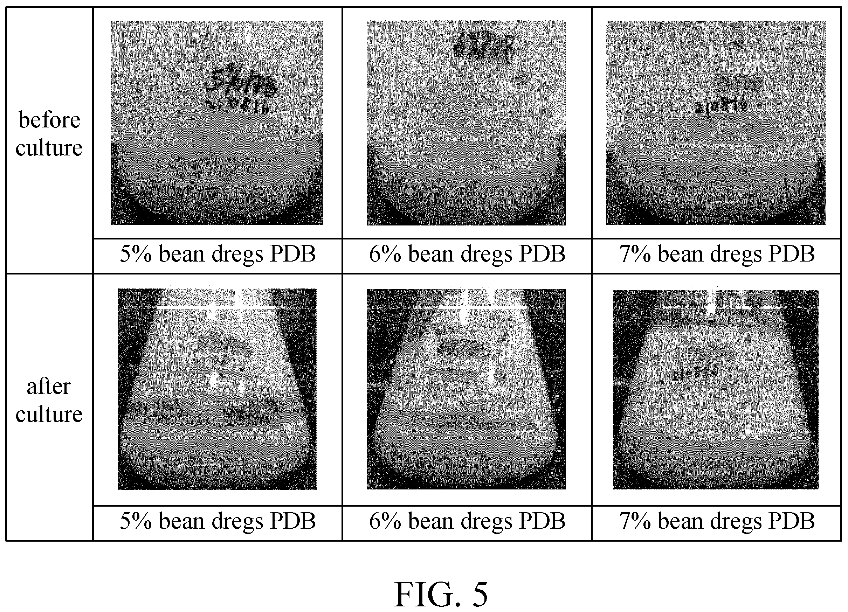

The tempeh bacteria of deposit number BCRC 31750 was used to carry out a comparative experiment. The tempeh bacteria BCRC 31750 was inoculated to PDB broth (Potato dextrose broth, STBIO MEDIA) containing 0%, 5%, 6% and 7% (w/v%) bean dregs respectively using an inoculation loop. The fermentation culture was performed at 30°C and 120 rpm for 4 days. FIG. 5 shows comparison of photographs taken before and after culture of the strain Tp-1 in PDB medium containing different proportions of bean dregs.

In addition, after the culture was completed, the culture medium was centrifuged at 20°C and 9900 rpm for 40 minutes, and the supernatant and the precipitate were stored separately. The precipitate was freeze-dried for 2 days and ground into powder, and the crude protein, crude fat, carbohydrate, water-soluble dietary fiber and water-insoluble dietary fiber content of the fermented product were analyzed. The measurement methods were as described in Example 4. The results are shown in Table 2.

**Table 2**

| | 0% bean dregs /PDB | 5% bean dregs /PDB | 6% bean dregs /PDB | 7% bean dregs /PDB |
|---|---|---|---|---|
| Crude protein (%) | 22.78±0.1 | 22.75±0.18 | 22.85±0.12 | 22.63±0.22 |
| Crude fat (%) | 14.78±0.4 | 13.51±0.59 | 12.23±1.69 | 12.48±0.12 |
| Carbohydrate (%) | 55.45±0.35 | 54.72±0.39 | 55.52±1.71 | 54.56±1.72 |
| Water-soluble dietary fiber (%) | 1.72±0.95 | 3.4±0.2 | 3.38±0.21 | 3.35±0.12 |
| Water-insoluble dietary fiber (%) | 21.70±1.80 | 18.4±0.24 | 18.27±0.18 | 18.26±1.04 |

According to the results of Table 2, after BCRC 31750 tempeh bacteria was inoculated in the medium of containing different proportions of bean dregs (0%, 5%, 6%, 7% (w/v%)) and fermented for 96 hours, the crude protein content of the medium containing bean dregs did not increase significantly. Compared with the group without bean dregs, the glycolysis in the groups containing bean dregs was not significant, while the water-soluble dietary fiber was slightly increased by about 1.6%. Comparing the results of Table 1 and Table 2, it can be seen that compared with BCRC 31750 tempeh bacteria, the novel *Rhizopus microsporus* strain Tp-1 provided by the present disclosure has the effects to improve the protein and water-soluble dietary fiber content of the fermentation product, and to improve the glycolysis conversion rate.

### Example 5: The physical property analysis of the fermentation product of novel Rhizopus microsporus strain

The strain Tp-1 was inoculated to different bean dregs and carried out solid-state fermentation to produce bean-dreg tempeh and the physical properties of the bean-dreg tempeh were measured with a texture analyzer (Horn Instruments co., ltd., model: UniversalTA). The bean source of bean-dreg tempeh A, B and E was provided by Taiwan Taoyuan Tofu Association (imported soybeans); the bean source of bean-dreg tempeh X was provided by PEACE VEGAN CO., LTD. (Taiwan organic soybeans).

The solid-state fermentation process was performed on the bean dregs of above-mentioned different sources. First, 1 kg of bean dregs was taken, dried in the oven at 60°C to 65°C for 24 hours, and sterilized with steam. After being cooled to room temperature, about 10 to 20 grams of Tp-1 strain powder or bacterial liquid was sprinkled into the bean dregs, stirred evenly, and then placed in a 30°C fermentation chamber to obtain a fermented product after 24 to 48 hours. A 2 cm³ square of the fermentation product was taken as a sample for texture analyzer measurement, and its physical properties such as hardness, springiness, chewiness, gumminess, cohesiveness, and resilience were analyzed.

The data of texture analyzer of the bean-dreg tempeh A (sample 1), bean-dreg tempeh B (sample 2), bean-dreg tempeh E (sample 3), and bean-dreg tempeh X (sample 4) of the different sources produced as described above are shown in Table 3. In addition, the data was compared with commercially available soybean tempeh (purchased from XiangHehJia-Biotech CO., LTD.) (sample 5), commercially available wheat TVP (provided by GOLDENCROPS CORPORATION) (sample 6), commercially available pea TVP (provided by GOLDENCROPS CORPORATION) (sample 7).

**Table 3**

| Sampl e | Hardness (N) | Springine ss (Nm) | Chewines s (N) | Gummine ss (N) | Cohesivene ss | Resilienc e |
|---|---|---|---|---|---|---|
| 1 | 8.39±0.48 | 0.25±0.01 | 0.73±0.0 2 | 2.90±0.17 | 0.34±0.01 | 0.15±0.0 1 |
| 2 | 7.08±0.59 | 0.30±0.03 | 0.80±0.0 6 | 2.69±0.16 | 0.38±0.01 | 0.16±0.0 1 |
| 3 | 7.71±0.30 | 0.27±0.03 | 0.77±0.1 1 | 2.83±0.12 | 0.37±0.02 | 0.16±0.0 1 |
| 4 | 15.54±0.7 4 | 0.30±0.04 | 1.76±0.3 4 | 5.79±0.36 | 0.37±0.04 | 0.18±0.0 2 |
| 5 | 24.08±3.4 5 | 0.36±0.03 | 3.01±0.8 4 | 8.23±1.79 | 0.34±0.05 | 0.17±0.0 2 |
| 6 | 10.92±5.8 6 | 0.55±0.03 | 4.53±2.5 0 | 8.16±4.34 | 0.75±0.02 | 0.44±0.0 9 |
| 7 | 8.14±1.04 | 0.68±0.02 | 4.15±0.8 3 | 6.08±1.13 | 0.74±0.05 | 0.47±0.0 1 |

As shown in the results of Table 3, the hardness of the fermented product (bean-dreg tempeh) produced by the fermentation of the strain Tp-1 was comparable to that of pea TVP, while other parameter values were lower. It can be seen that the texture of bean-dreg tempeh is relatively soft and easy to chew. With appropriate seasoning and simple hygienic processing, it can be used as a ready-to-eat conditioning package, which is quite suitable for providing high-protein nutrition to patients who need medical care or the elderly. Moreover, in the production of vegetable meat and semi-finished products for vegetarian food, bean-dreg tempeh can also serve as a natural protein raw material or an ingredient additive to increase the ingredient diversity or taste of the food.

## Claims

1. An isolated and purified novel *Rhizopus microsporus* strain, with deposit number DSM 34400.

2. A method for preparing a fermented food by using a *Rhizopus microsporus* strain, comprising:
providing an isolated and purified *Rhizopus microsporus* strain, wherein its deposit number is DSM 34400; and
inoculating the isolated and purified *Rhizopus microsporus* strain to a substrate for fermentation to form a fermented food, wherein the substrate comprises a legume, a processing residue of a legume, or a combination thereof.

3. The method according to claim 2, wherein the substrate is the processing residue of the legume.

4. The method according to claim 2 or claim 3, wherein a processing is performed on the legume to obtain the processing residue of the legume, and the processing comprises a cooking step, a pressing step, a drying step, a grinding step, or a combination thereof.

5. The method according to any one of claims 2 to 4, wherein the processing residue of the legume comprises dregs of the legume.

6. The method according to any one of claims 2 to 5, wherein the legume comprises soybean, black bean, red bean, mung bean, edamame, kidney bean, red kidney bean, pinto bean, pea, pigeon pea, cowpea, broad bean, chickpea, lentil, hyacinth bean, peanut, lupine, grass pea, carob, or a combination thereof.

7. The method according to any one of claims 2 to 6, wherein the fermentation comprises a solid-state fermentation process, a liquid-state fermentation process or a combination thereof, wherein a temperature of the fermentation is between 20°C and 40°C, and/or wherein the fermentation is performed for between 20 hours and 50 hours.

8. The method according to any one of claims 2 to 7, wherein the isolated and purified *Rhizopus microsporus* strain is provided in the form of a freeze-dried powder or a bacterial broth.

9. The method according to any one of claims 2 to 8, wherein the fermented food comprises a tempeh, a textured vegetable protein (TVP) additive or a functional drink.

10. The method according to any one of claims 2 to 9, wherein after inoculating the isolated and purified *Rhizopus microsporus* strain to the substrate for fermentation, the method further comprises performing a uniaxial wet extrusion process or a biaxial wet extrusion process on the substrate to form the fermented food.

11. A fermented food, which is formed by fermentation of a substrate by using an isolated and purified *Rhizopus microsporus* strain, wherein the deposit number of the isolated and purified *Rhizopus microsporus* strain is DSM 34400;
wherein the substrate comprises a legume, a processing residue of a legume, or a combination thereof; and
wherein the fermented food has a hardness of 6.48 N to 27.54 N, or a springiness of 0.24 Nm to 0.70 Nm, or a chewiness of 0.70 N to 5.00 N, or a gumminess of 2.7 N to 10.05 N, or a cohesiveness of 0.32 to 0.80, or a resilience of 0.14 to 0.50.

12. The fermented food according to claim 11, wherein protein content of the fermented food is increased by 2% to 10% compared to protein content of an unfermented substrate, the protein content of the fermented food is between 22% and 28%, and water-soluble dietary fiber content of the fermented food is between 2.70% and 5.67%.

13. The fermented food according to claim 11 or claim 12, wherein the processing residue of the legume comprises dregs of the legume.

14. The fermented food according to any one of claims 11 to 13, wherein the legume comprises soybean, black bean, red bean, mung bean, edamame, kidney bean, red kidney bean, pinto bean, pea, pigeon pea, cowpea, broad bean, chickpea, lentil, hyacinth bean, peanut, lupine, grass pea, carob, or a combination thereof.

15. The fermented food according to any one of claims 11 to 14, wherein the fermented food comprises a tempeh, a textured vegetable protein (TVP) additive or a functional drink.

## Patentansprüche

1. Isolierter und gereinigter neuartiger *Rhizopus-mikrosporus-Stamm,* mit Hinterlegungsnummer DSM 34400.

2. Verfahren zur Herstellung eines fermentierten Lebensmittels unter Verwendung eines *Rhizopus-mikrosporus-*Stamms, umfassend:
Bereitstellen eines isolierten und gereinigten *Rhizopus-mikrosporus-Stamms,* wobei seine Hinterlegungsnummer DSM 34400 ist; und
Animpfen eines Substrats für Fermentation mit dem isolierten und gereinigten *Rhizopus-mikrosporus-*Stamm*,* um ein fermentiertes Lebensmittel zu bilden, wobei das Substrat eine Leguminose, einen Verarbeitungsrückstand von einer Leguminose, oder eine Kombination davon umfasst.

3. Verfahren gemäß Anspruch 2, wobei das Substrat der Verarbeitungsrückstand von der Leguminose ist.

4. Verfahren gemäß Anspruch 2 oder Anspruch 3, wobei ein Verarbeiten an der Leguminose durchgeführt wird, um den Verarbeitungsrückstand von der Leguminose zu erhalten, und das Verarbeiten einen Kochschritt, einen Pressschritt, einen Trockenschritt, einen Mahlschritt, oder eine Kombination davon umfasst.

5. Verfahren gemäß einem der Ansprüche 2 bis 4, wobei der Verarbeitungsrückstand von der Leguminose Pülpe von der Leguminose umfasst.

6. Verfahren gemäß einem der Ansprüche 2 bis 5, wobei die Leguminose Sojabohne, Schwarze Bohne, Rote Bohne, Mungbohne, Edamame, Kidneybohne, rote Kidneybohne, Pintobohne, Erbse, Straucherbse, Kuhbohne, Ackerbohne, Kichererbse, Linse, Hyazinth-Bohne, Erdnuss, Lupine, Saat-Platterbse, Johannisbrot, oder eine Kombination davon umfasst.

7. Verfahren gemäß einem der Ansprüche 2 bis 6, wobei die Fermentation ein Festphasen-Fermentationsverfahren, ein Flüssigphasen-Fermentationsverfahren oder eine Kombination davon umfasst, wobei eine Temperatur der Fermentation zwischen 20°C und 40°C liegt, und/oder wobei die Fermentation zwischen 20 Stunden und 50 Stunden lang durchgeführt wird.

8. Verfahren gemäß einem der Ansprüche 2 bis 7, wobei der isolierte und gereinigte *Rhizopus-mikrosporus-*Stamm in der Form eines gefriergetrockneten Pulvers oder eines Bakterien-Nährmediums bereitgestellt wird.

9. Verfahren gemäß einem der Ansprüche 2 bis 8, wobei das fermentierte Lebensmittel ein Tempeh, ein texturiertes Pflanzenprotein (TVP)-Additiv oder ein funktionelles Getränk umfasst.

10. Verfahren gemäß einem der Ansprüche 2 bis 9, wobei nach dem Animpfen des Substrats für Fermentation mit dem isolierten und gereinigten *Rhizopus-mikrosporus-*Stamm das Verfahren ferner Durchführen eines uniaxialen Feuchtextrusionsverfahrens oder eines biaxialen Feuchtextrusionsverfahrens an dem Substrat umfasst, um das fermentierte Lebensmittel zu bilden.

11. Fermentiertes Lebensmittel, welches durch Fermentation eines Substrats unter Verwendung eines isolierten und gereinigten *Rhizopus-mikrosporus-Stamms* gebildet wird, wobei die Hinterlegungsnummer des isolierten und gereinigten *Rhizopus-mikrosporus-Stamms* DSM 34400 ist;
wobei das Substrat eine Leguminose, einen Verarbeitungsrückstand von einer Leguminose, oder eine Kombination davon umfasst; und
wobei das fermentierte Lebensmittel eine Härte von 6,48 N bis 27,54 N, oder eine Elastizität von 0,24 Nm bis 0,70 Nm, oder eine Kaubarkeit von 0,70 N bis 5,00 **N,** oder eine Gummiartigkeit von 2,7 N bis 10,05 N, oder eine Kohäsionskraft von 0,32 bis 0,80, oder eine Rückprallelastizität von 0,14 bis 0,50 aufweist.

12. Fermentiertes Lebensmittel gemäß Anspruch 11, wobei ein Proteingehalt von dem fermentierten Lebensmittel um 2% bis 10% verglichen mit einem Proteingehalt von einem nicht fermentierten Substrat erhöht ist, wobei der Proteingehalt von dem fermentierten Lebensmittel zwischen 22% und 28% liegt, und ein wasserlöslicher Ballaststoff-Gehalt von dem fermentierten Lebensmittel zwischen 2,70% und 5,67% liegt.

13. Fermentiertes Lebensmittel gemäß Anspruch 11 oder Anspruch 12, wobei der Verarbeitungsrückstand von der Leguminose Pülpe von der Leguminose umfasst.

14. Fermentiertes Lebensmittel gemäß einem der Ansprüche 11 bis 13, wobei die Leguminose Sojabohne, Schwarze Bohne, Rote Bohne, Mungbohne, Edamame, Kidneybohne, rote Kidneybohne, Pintobohne, Erbse, Straucherbse, Kuhbohne, Ackerbohne, Kichererbse, Linse, Hyazinth-Bohne, Erdnuss, Lupine, Saat-Platterbse, Johannisbrot, oder eine Kombination davon umfasst.

15. Fermentiertes Lebensmittel gemäß einem der Ansprüche 11 bis 14, wobei das fermentierte Lebensmittel ein Tempeh, ein texturiertes Pflanzenprotein (TVP)-Additiv oder ein funktionelles Getränk umfasst.

## Revendications

1. Nouvelle souche de *Rhizopus microsporus* isolée et purifiée, avec le numéro de dépôt DSM 34400.

2. Procédé de préparation d'un aliment fermenté en utilisant une souche de *Rhizopus microsporus,* comprenant :
la fourniture d'une souche de *Rhizopus microsporus* isolée et purifiée, dans lequel sont numéro de dépôt est DSM 34400 ; et
l'inoculation de la souche de *Rhizopus microsporus* isolée et purifiée à un substrat pour la fermentation afin de former un aliment fermenté, dans lequel le substrat comprend une légumineuse, un résidu de traitement d'une légumineuse ou une combinaison de ceux-ci.

3. Procédé selon la revendication 2, dans lequel le substrat est le résidu de traitement de la légumineuse.

4. Procédé selon la revendication 2 ou la revendication 3, dans lequel un traitement est effectué sur la légumineuse pour obtenir le résidu de traitement de la légumineuse, et le traitement comprend une étape de cuisson, une étape de pressage, une étape de séchage, une étape de broyage ou une combinaison de celles-ci.

5. Procédé selon l'une quelconque des revendications 2 à 4, dans lequel le résidu de traitement de la légumineuse comprend des marcs de la légumineuse.

6. Procédé selon l'une quelconque des revendications 2 à 5, dans lequel la légumineuse comprend le soja, le haricot noir, le haricot rouge, le haricot mungo, l'edamame, le haricot rouge commun, le haricot pinto, le pois, le pois d'Angole, le niébé, la fève, le poids chiche, la lentille, le haricot jacinthe, l'arachide, le lupin, le pois carré, la caroube ou une combinaison de ceux-ci.

7. Procédé selon l'une quelconque des revendications 2 à 6, dans lequel la fermentation comprend un processus de fermentation à l'état solide, un processus de fermentation à l'état liquide ou une combinaison de ceux-ci, dans lequel une température de la fermentation est comprise entre 20 °C et 40 °C, et/ou dans lequel la fermentation est réalisée pendant une durée comprise entre 20 heures et 50 heures.

8. Procédé selon l'une quelconque des revendications 2 à 7, dans lequel la souche de *Rhizopus microsporus* isolée et purifiée est fournie sous la forme d'une poudre lyophilisée ou d'un bouillon bactérien.

9. Procédé selon l'une quelconque des revendications 2 à 8, dans lequel l'aliment fermenté comprend un tempeh, un additif de protéine végétale texturée (TVP) ou une boisson fonctionnelle.

10. Procédé selon l'une quelconque des revendications 2 à 9, dans lequel, après l'inoculation de la souche de *Rhizopus microsporus* isolée et purifiée au substrat pour la fermentation, le procédé comprend en outre l'exécution d'un processus d'extrusion humide uniaxiale ou d'un processus d'extrusion humide biaxiale sur le substrat pour former l'aliment fermenté.

11. Aliment fermenté, qui est formé par la fermentation d'un substrat en utilisant une souche de *Rhizopus microsporus* isolée et purifiée, dans lequel le numéro de dépôt de la souche de *Rhizopus microsporus* isolée et purifiée est DSM 34400 ;
dans lequel le substrat comprend une légumineuse, un résidu de traitement d'une légumineuse ou une combinaison de ceux-ci ; et
dans lequel l'aliment fermenté présente une dureté de 6,48 N à 27,54 N, ou une élasticité de 0,24 Nm à 0,70 Nm, ou une masticabilité de 0,70 N à 5,00 N, ou une gommosité de 2,7 N à 10,05 N, ou une cohésion de 0,32 à 0,80, ou une résilience de 0,14 à 0,50.

12. Aliment fermenté selon la revendication 11, dans lequel la teneur en protéines de l'aliment fermenté est augmentée de 2 % à 10 % par rapport à la teneur en protéines d'un substrat non fermenté, la teneur en protéines de l'aliment fermenté est comprise entre 22 % et 28 %, et la teneur en fibres alimentaires hydrosolubles de l'aliment fermenté est comprise entre 2,70 % et 5,67 %.

13. Aliment fermenté selon la revendication 11 ou la revendication 12, dans lequel le résidu de traitement de la légumineuse comprend des marcs de la légumineuse.

14. Aliment fermenté selon l'une quelconque des revendications 11 à 13, dans lequel la légumineuse comprend le soja, le haricot noir, le haricot rouge, le haricot mungo, l'edamame, le haricot rouge commun, le haricot pinto, le pois, le pois d'Angole, le niébé, la fève, le poids chiche, la lentille, le haricot jacinthe, l'arachide, le lupin, le pois carré, la caroube ou une combinaison de ceux-ci.

15. Aliment fermenté selon l'une quelconque des revendications 11 à 14, dans lequel l'aliment fermenté comprend un tempeh, un additif de protéine végétale texturée (TVP) ou une boisson fonctionnelle.
